# EUROPÄISCHE PATENTSCHRIFT

(11) **EP 0 534 386 B1**
(45) Veröffentlichungstag und Bekanntmachung des Hinweises auf die Patenterteilung: **17.12.1997**
(21) Anmeldenummer: 92116250.9
(22) Anmeldetag: 23.09.1992
(51) Int. Cl.: A61M 1/16, B01D 63/02

(54) **Medizinische Einrichtung zum Stoffaustausch zwischen zwei Medien durch Membrane**
Medical device for mass exchange between two media through a membrane
Dispositif médical pour l'échange de matière entre deux milieux à travers une membrane

(30) Priorität: 24.09.1991 DE 4131795
(43) Veröffentlichungstag der Anmeldung: 31.03.1993
(73) Patentinhaber: Oedekoven, Bernward, 6351 HX Bocholtz (NL)
(72) Erfinder: Oedekoven, Bernward, NL-6351 HX Bocholtz (NL); Mottaghy, Khosrow, Prof. Dr., W-5100 Aachen (DE); Kashefi-Khorasani, Ahmad Ali, Dipl.-Ing., W-5100 Aachen (DE); Herdlicka, Wolfgang, Jr., W-8080 Fürstenfeldbruck (DE)
(74) Vertreter: Kern, Ralf M., Dipl.-Ing.

(56) Entgegenhaltungen:
- EP-A- 0 183 250
- EP-A- 0 299 381
- EP-A- 0 315 420
- WO-A-89/11884
- DE-A- 3 812 987
- FR-A- 2 267 138
- MED. PROGRESS THROUGH TECHNOLOGY Bd. 4, Nr. 3, 1976, BERLIN Seiten 139 - 145 ZINGG ET AL. 'improving efficiency of tubular membrane oxygenator'

## Beschreibung

Bekannt sind eine Vielzahl von Oxygenatoren oder Dialyse-Einrichtungen, bei denen über bzw. durch Membrane hindurch ein Stoffaustausch, beispielsweise bei Oxygenatoren von Sauerstoff in das Blut hinein und Kohlendioxid aus dem Blut eines Probanten erfolgt.

Neuere Ausführungen eines solchen Oxygenators bestehen aus einem Zylinder, an dessen beiden Stirnenden mittels Verteilerköpfen eine Aufteilung des einen Mediums, z. B. des Bluts, in eine Vielzahl dünner Kapillarschläuche (z. B. mit weniger als 1 mm Durchmesser) aus gasdurchlässigem, porenfreiem Material (vorzugsweise aus Kunststoff) erfolgt, welche den Zylinderinnenraum, welcher mit dem Austauschmedium (z. B. Sauerstoff oder sauerstoffangereichertes Gas) gefüllt ist bzw. durchflossen wird und diesen, ggf. in Gegenrichtung zum Blut, bis zur anderen Stirnfläche durchziehen. Am Ende einer bestimmten Kapillarlänge, welche multipliziert mit der Anzahl der Kapillaren die Austauschfläche bestimmt, wird das Blut mit den verbesserten Gas-Partialdrücken aus den Kapillar-Schläuchen durch den dort befindlichen Verteilerkopf wieder zu einem Strom vereinigt. Im Prinzip gleichartig funktionieren Oxygenatoren, deren Blutführung außerhalb der Kapillaren, die in gewobener, wendelartiger oder anderer Anordnung vorliegen können, geschieht, während der Gasfluß durch die Kapillaren hindurch geleitet wird. Analog wie beim oben beschriebenen innendurchströmten Kapillar-Oxygenator wird dieses Prinzip bei der Dialyse für den entsprechend anderen Stoffaustausch eingesetzt.

Diese an sich bewährten Geräte haben jedoch den Nachteil, daß sie einerseits vergleichsweise großvolumig, starr und schwer ausgebildet sind und damit ein großes zusätzliches Totvolumen an Blut aufweisen, welches nicht am Gasaustausch beteiligt ist und für ihren Einsatz vorherige oder während der Behandlung erfolgende Blut- und Plasmainfusionen (Blutkonserven) bedingen, die den Körper des Probanten in vielfacher Hinsicht ungünstig beeinflussen können.

Unvermeidlicherweise wird dieses Totvolumen noch durch die blutführenden Schläuche vom Patienten zur Herz-Lungen-Maschine und von dieser zurück zum Patienten ganz erheblich vergrößert. In den klinischen Routine-Einsätzen handelt es sich dabei um mehrere Meter Schlauchmaterial und demzufolge mehrere 100 ml Blutvolumen. Die zusätzlich zu den reinen Schlauchverbindungen blutflußmäßig vor und hinter der Blutpumpe, z. B. einer oder mehrerer Rollerpumpen angeordneten Blutreservoire haben allein je Reservoir häufig ein Blutvolumen von 1,0 - 1,5 Ltr., welches bei modernen Anordnungen zwar erheblich verkleinert werden konnte, aber trotzdem immer noch zu groß ist.

Ein weiterer Nachteil der vorgenannten bisherigen Oxygenatoren liegt darin, daß sie wegen ihrer aufwendigen Konstruktion volumenmäßig keinerlei Unterschiede erlauben, beispielsweise keine flexible Anpassung an Patienten mit geringerem Blutvolumen oder GasaustauschBedarf oder z. B. für Kleinstkinder und Kinder verschiedener Alters- und damit Gewichtsstufen, so daß z. B. der Oxygenatoreinsatz bei Früh- und Neugeborenen einem - nicht erwünschten - Blutaustausch zumindest nahekommt. Gerade bei Kleinkindern entsprechen derartige Eingriffe nicht einer erwünschten Behandlung, da für den Eingriff mit herkömmlichen Oxygenatoren Fremdblutvolumia benötigt werden, die das 2- bis 3-fache des Eigenblutanteils der Kinder ausmachen können.

Aus dem Artikel MED.PROGR.TECHNOL.4, Seiten 139-195; Wezingg et al. "Improving the Efficiency of a Tubular Membran Oxygenator" ist bereits eine medizinische Einrichtung zum Stoff- und Gasaustausch zwischen zwei Medien durch Membrane unter Verwendung einer Vielzahl von dünnen Kapillarschläuchen innerhalb einer diese umhüllenden Wandung bekannt, wobei die Kapillarschläuche innerhalb einer extrakorporalen blutleitenden Verbindung mit einem Patienten anordenbar sind.

Diese medizinische Einrichtung ist einstückig.

Dieser Nachteil wird durch eine medizinische Einrichtung nach der EP 0 315 420 vermieden, bei dem die medizinische Einrichtung aus verschiedenen Modulen besteht. Bei diesen Modulen sind die Zuleitungen zu dem Raum zwischen den Kapillaren seitlich angebracht, so daß der Raum zwischen den Kapillaren nicht als blutleitender Raum ausgelegt sein kann. Daher muß das Blut durch die Kapillaren fließen, wobei ein erhöhter Druck erforderlich ist. Außerdem hat diese Anordnung den Nachteil, daß die Kapillarschläuche nicht beliebig dünn gemacht werden können, ohne daß unverhältnismäßige Scherkräfte auf die Erethrozyten usw. ausgeübt werden. Daher kann das Austauschvolumen nicht beliebig vergrößert werden.

Aufgabe der Erfindung ist es daher, eine aus Modulen bestehende medizinische Einrichtung bereitzustellen, bei der diese Nachteile vermieden sind. Diese Aufgabe wird bei einer gattungsgemäßen medizinischen Einrichtung durch die Merkmale des kennzeichnenden Teils von Anspruch 1 gelöst.

Bevorzugte Ausführungsbeispiele der Erfindung sind in den Unteransprüchen dargestellt.

Die Erfindung ist nachstehend in mehreren Ausführungsbeispielen näher - in weitgehend schematischer Darstellung - beschrieben.

Es zeigt
Fig. 1
   eine perspektivische, teilweise weggebrochene grundsätzliche Ausbildung der Erfindung,
Fig. 2
   einen vergrößerten Teilquerschnitt einer grundsätzlichen Ausbildung der erfindungsgemäßen Einrichtung mit Kapillaren innerhalb eines Schlauchs,
Fign. 3a und 3b
   vergrößerte Teilquerschnitte zweier verschiedener Formen von Anschluß-Kopfstücken,
Fig. 4 und 5
   zwei schematische Anordnungen von erfindungsgemäßen Schlauchmodulen in hintereinander (Fig. 4) und paralleler Anordnung zueinander,
Fig. 6a, 6b und 6c
   teilschnittmäßig dargestellte verschiedene Anordnungen der Kapillaren innerhalb des Schlauch-Innenraums,
Fig. 7 und 8
   zwei weitere Ausführungen unterschiedlicher Führung der Kapillarbündel innerhalb eines erfindungsgemäßen schlauchartigen Gefäßes,

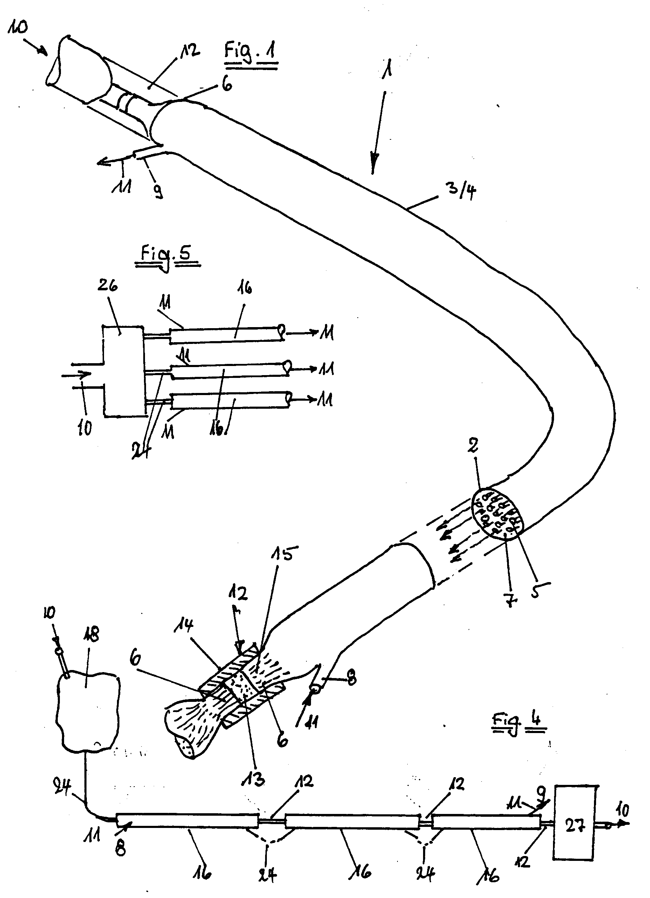

Die in Fig. 1 dargestellte medizinische Stoffaustausch-Einrichtung 1 weist innerhalb der äußeren Wandung 2 eines Gefäßes 3, in Fig. 1 eines Schlauches 4 eine Vielzahl von Kapillaren 5 auf, deren Enden 6 mittels eines Anschlußkopfes 15 wieder zusammengeführt sind. Das nach der Verdrängung durch die Kapillaren übrige Innenvolumen des Schlauch-Innenraums ist mit gesonderten Anschlüssen, d. h. einem Einlaß 8 und Auslaß 9 versehen.

Zwischen dem im Inneren der Kapillaren 5 befindlichen einen Medium 10 (Fig. 2) und dem im Innen-Volumen 7 befindlichen anderen Medium 11 erfolgt der Austausch von O₂ und CO₂ durch Diffusion durch die dünnen Wände der Kapillaren 5 z. B. bei der Verwendung von Silikon-Gummi bzw. durch Konvektion bei der Verwendung mikroporöser Kapillaren wie sie z. B. aus Polypropylen hergestellt werden können.

In den nachstehenden Ausführungsformen kann entweder das zu behandelnde Blut als das eine Medium 10 durch die Kapillaren 5 fließen und der Sauerstoff bzw. das ausgeschiedene CO₂ als das andere Medium durch das Innenvolumen 7 des Schlauchs 4 strömen oder umgekehrt, so daß die angegebenen Pfeile stets nur eine Alternativ-Lösung angeben. Ebenso können die beiden Medien 10, in gleicher Richtung oder in Gegenstromrichtung fließen, so daß die angegebenen Pfeilrichtungen ebenfalls grundsätzlich nur eine von zwei Möglichkeiten angeben, obwohl die Verwendung in Gegenstromrichtung oft die vorteilhaftere Lösung darstellen kann. Damit sind Einlaß und Auslaß 9 auch umgekehrt zu verstehen.

Auch die Durchmesser von Kapillaren 5 und des Schlauches 4 sowie die Wandstärke der Kapillaren und ihre Anzahl innerhalb eines Schlauchdurchmessers ist eine Optimierungsfrage und abhängig von den Gasaustausch-Leistungsanforderungen an das Gerät sowie die notwendigen Blutflußraten. Da es sich bei dem einen Medium 10 um Blut handelt, sind zu hohe Schubspannungen zu vermeiden, was bei der konstruktiven Auslegung des Gerätes z. B. bei der Wahl der Kapillardurchmesser, Kapillaranzahl, Kapillarlänge sowie der Blutführung durch die bzw. außerhalb der Kapillaren 5 Beachtung findet.

In Fig. 1 bzw. überhaupt nachstehend wird Blut als das eine Medium 10 duch die Kapillaren 5 geleitet, welche vom anderen durch das als anderes Medium 11 im Innenvolumen 7 umströmt werden, wobei das Medium 11 durch an den Enden des Schlauches 4 je einen Einlaß 8 und einen Auslaß 9 durch den Schlauch strömt. Die Optimierung der Strömungscharakteristik des Mediums 11 läßt erfindungsgemäß auch zu, daß im Schlauch 4 mehrere, gegenüberliegende Ein- 8 und Auslässe 9 angeordnet sind, wobei das Medium 11 im "Cross-Flow" also quer zur Fließrichtung des Mediums 10 durchgesaugt oder durchgepreßt wird.

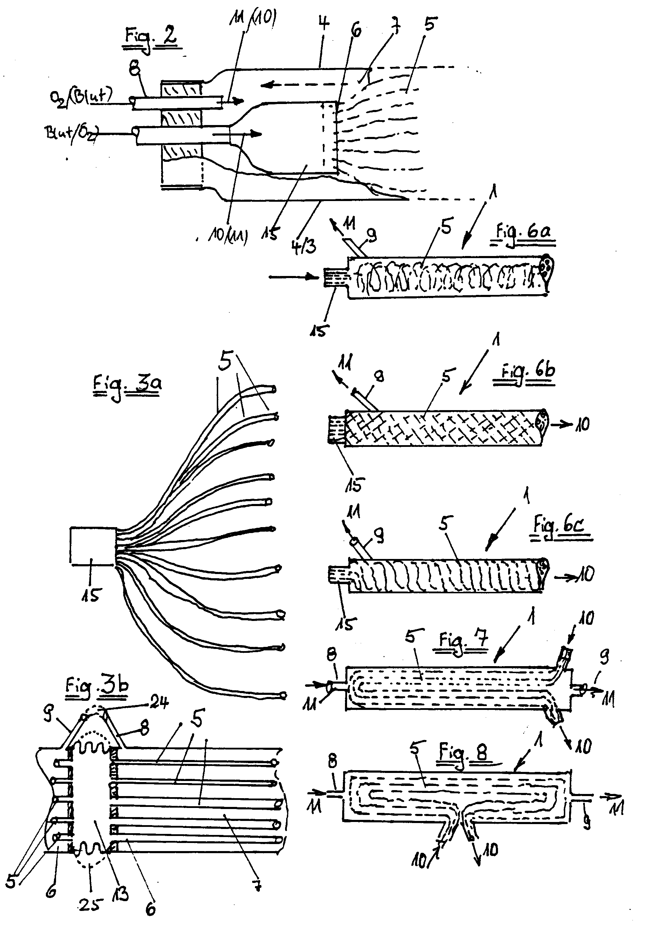

Die als Schlauchstück 4 ausgeführte Einrichtung 1 besitzt an ihren Enden 6 je einen Kapillar-Anschlußkopf 15 z. B. Fig. 3a, welcher über eine Kupplung 12 mit Verbindungsmanschette 14 und einem Übergangsraum 13 mit einer weiteren Einrichtung 1 verbindbar ist (z. B. Fig. 1 und Fig. 3b).

Der Übergangsraum 13 kann mit seiner Wandung 25 auch mit druckausgleichender Funktion als flexible Wandung ausgebildet sein, beispielsweise balgartig gemäß Fig. 3b. Eine Verbindung 24 (s. Fig. 3b und 4) verbindet die Innenräume 7 zweier mittels geeigneter Schnellverbindungskupplung aneinandergesetzter Schlaucheinrichtungen 1 in Form von Schlauchmodulen 16 die auch zu mehreren zusammengeschaltet werden können. Gemäß Fig. 5 können solche Schlauchmodule 16 in einer Ausführungsform über einen Verteilerkopf 26 parallel zueinander geschaltet angeordnet sein. Im Sinne von Fig. 4 bilden Module 16 in Verbindung mit einem flexiblen Blut-Reservoirbeutel 18 in Serie hintereinander geschaltet die Schlauchverbindung zu einer Blutpumpe 27.

Zur Vergrößerung der Stoffübergangsfläche zwischen Kapillaren und Innenraum 7 können die Kapillaren 5 gemäß Fig. 6a in Wendeln oder Spiralen, gemäß Fig. 6b in netz- oder schwammförmiger Anordnung, gemäß Fig. 6c in miteinander verdrehter Form im Innenraum 7 angeordnet sein. In den Fig. 7 und 8 sind die Kapillaren 5 in Schleifen innerhalb eines Schlauchmoduls 16 vorgesehen, und zwar mit an einer Seite zusammengelegtem Ein- und Auslaß für das Blut 10.

## Patentansprüche

1. Medizinische Einrichtung zum Stoff- und Gasaustausch zwischen Blut und einem anderen Medium, z.B. Gas, mit einer Vielzahl von dünnen Kapillarschläuchen (5) innerhalb einer diese umhüllenden Wandung (4), wobei jeweils mit Kapillaren (5) durchzogene Teilstücke der Einrichtung als Module zusammen kombinierbar sind, dadurch gekennzeichnet, daß die Module als Schlauchmodule (16) ausgelegt sind und jedes Schlauchmodul (16) an beiden Enden jeweils eine Kupplung (12) aufweist, wobei die Zuleitungen (10, 11) sowohl zum Innenraum der Kapillaren (5) als auch zum Zwischenraum zwischen den Kapillaren innerhalb der Kupplung (12) axial angeordnet sind, so daß der Zwischenraum zwischen den Kapillaren (5) als blutführender Raum ausgelegt sein kann.

2. Medizinische Einrichtung nach Anspruch 1, dadurch gekennzeichnet, daß die Kapillarschläuche (5) innerhalb der umhüllenden Gefäß-Außenwandung der Teilstücke in Oberflächen vergrößerter Anordnung, z.B. schlingen-, wendel- oder knäuelartig, enthalten sind.

3. Medizinische Einrichtung nach einem der vorhergehenden Ansprüche, dadurch gekennzeichnet, daß alle blutkontaktierenden Oberflächen mit Heparin oder anderen Antikoagulantien beschichtet sind, z.B. in Form einer kovalenten Bindung, oder durch andere oberflächenveredelnden Maßnahmen blutfreundlicher und blutgerinnungshemmend bearbeitet sind.

4. Medizinische Einrichtung nach einem der vorhergehenden Ansprüche 2 oder 3, dadurch gekennzeichnet, daß nur ein Schlauchmodul (16) oder sehr wenige Schlauchmodule vorhanden ist bzw. sind, wobei jedes Schlauchmodul (16) so ausgelegt ist, daß sein Blutvolumen wesentlich unter dem Eigenblutvolumen eines Früh- oder Neugeborenen liegt.

## Claims

1. Medical device for mass exchange and gas exchange between blood and another medium, e.g. gas, having a large number of thin capillary tubes (5) inside a wall (4) which encloses these, it being possible for component parts of the device which each have capillaries (5) passing through them to be combined together as modules, characterized in that the modules are designed as tubular modules (16) and each tubular module (16) has a coupling (12) at both of its ends, and the admission lines (10, 11) both to the interior of the capillaries (5) and also to the interspace between the capillaries are arranged axially within the coupling (12) so that the interspace between the capillaries (5) can be designed as a blood-conveying space.

2. Medical device according to Claim 1, characterized in that the capillary tubes (5) are contained inside the enclosing vessel wall of the component parts in surfaces of enlarged configuration, e.g. in loops, coils or tangles.

3. Medical device according to one of the preceding claims, characterized in that all surfaces coming into contact with blood are coated with heparin or other anticoagulants, e.g. in the form of a covalent bond, or are made more haemocompatible and anticoagulant by means of other surface-finishing measures.

4. Medical device according to one of the preceding Claims 2 and 3, characterized in that only one tubular module (16) or very few tubular modules is/are present, each tubular module (16) being designed so that its blood volume lies substantially below the intrinsic blood volume of a premature baby or neonate.

## Revendications

1. Appareil médical pour l'échange de matières et de gaz entre du sang et un autre milieu, par exemple du gaz, comprenant une pluralité de tubes capillaires minces (5) à l'intérieur d'une paroi (4) qui les entoure, des éléments partiels d'appareil, respectivement traversés par des capillaires (5), pouvant être combinés ensemble sous forme de modules, caractérisé en ce que les modules sont conçus sous forme de modules en tuyau (16), et chaque module en tuyau (16) comporte aux deux extrémités un accouplement respectif (12), les conduites d'amenée (10, 11) vers la chambre intérieure des capillaires (5) ainsi que vers la chambre intermédiaire entre les capillaires à l'intérieur de l'accouplement (12) étant agencées axialement, de sorte la chambre intermédiaire entre les capillaires (5) peut être conçue comme chambre pour le passage du sang.

2. Appareil médical selon la revendication 1, caractérisé en ce que les tubes capillaires (5) sont contenus à l'intérieur de la paroi extérieure de récipient des éléments partiels qui les entourent sous forme d'agencements à surface augmentée, par exemple en forme de boucles, d'hélices ou de tiges.

3. Appareil médical selon l'une des revendications précédentes, caractérisé en ce que toute les surfaces en contact avec le sang sont revêtues d'héparine ou d'autres produits anti-coagulants, par exemple sous la forme d'un composé covalent, ou sont traitées par d'autres opérations de finition de surface pour ménager le sang et empêcher la coagulation du sang.

4. Appareil médical selon l'une ou l'autre des revendications 2 et 3, caractérisé en ce qu'il n'est prévu qu'un seul module en tuyau (16) ou qu'un petit nombre de modules en tuyau, et chaque module en tuyau (16) est conçu de manière que son volume de sang est situé nettement au-dessous du volume de sang propre d'un nouveau-né ou d'un prématuré.
